(19) Europäisches Patentamt
European Patent Office
Office européen des brevets

(11) EP 3 269 701 B1

# (12) EUROPEAN PATENT SPECIFICATION

(45) Date of publication and mention of the grant of the patent:
**08.05.2019 Bulletin 2019/19**

(21) Application number: **16761700.0**

(22) Date of filing: **07.03.2016**

(51) Int Cl.:
***C07C 67/03*** (2006.01) ***C07C 67/54*** (2006.01)
***C07C 69/54*** (2006.01) ***C07B 61/00*** (2006.01)

(86) International application number:
**PCT/JP2016/056954**

(87) International publication number:
**WO 2016/143732 (15.09.2016 Gazette 2016/37)**

## (54) METHOD FOR PRODUCING (METH)ACRYLATE ESTER COMPOUNDS

VERFAHREN ZUR HERSTELLUNG VON (METH)ACRYLATESTERVERBINDUNGEN

PROCÉDÉ DE PRODUCTION DE COMPOSÉS D'ESTER DE (MÉTH)ACRYLATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.03.2015 JP 2015049344**

(43) Date of publication of application:
**17.01.2018 Bulletin 2018/03**

(73) Proprietor: **Kuraray Co., Ltd.**
**Okayama 710-0801 (JP)**

(72) Inventors:
- **TAKAHATA, Yusuke**
  **Kurashiki-shi**
  **Okayama 710-0801 (JP)**
- **KAJIYASHIKI, Tsuyoshi**
  **Kurashiki-shi**
  **Okayama 710-0801 (JP)**

(74) Representative: **Müller-Boré & Partner Patentanwälte PartG mbB**
**Friedenheimer Brücke 21**
**80639 München (DE)**

(56) References cited:
**JP-A- H06 340 584**
**JP-A- H07 133 252**
**JP-A- H07 133 252**
**JP-A- S55 143 935**
**JP-A- S55 143 935**
**JP-A- 2004 035 728**
**JP-A- 2004 091 613**
**US-A- 4 904 814**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### TECHNICAL FIELD

The present invention relates to a method for producing a (meth)acrylate ester compound of an alcohol compound having a tertiary hydroxyl group, in particular, to a method for producing a (meth)acrylate ester compound by transesterification of an alkyl (meth)acrylate with a polyhydric alcohol compound having a tertiary hydroxyl group and also having a primary hydroxyl group and/or a secondary hydroxyl group in the presence of an iron complex.

### BACKGROUND ART

As conventionally known in the art, the transesterification of methyl (meth)acrylate and an alcohol compound is performed using an iron complex as a catalyst. For example, Patent Literature 1 describes that methyl methacrylate is transesterified with an alcohol compound having a primary hydroxyl group or a secondary hydroxyl group in the presence of an iron catalyst to form the methacrylate ester compound of the alcohol compound having a primary hydroxyl group or a secondary hydroxyl group.

Patent Literature 2 describes that the transesterification of methyl (meth)acrylate with 3-methylbutane-1,3-diol in the presence of an iron complex affords 3-hydroxy-3-methylbutyl (meth)acrylate. Patent, Literature 3 discloses a transesterification of methyl methacrylate with a tertiary alcohol in the presence of a catalyst. The water content in said transesterification is more than 100 ppm . The catalyst comprises a combination of an alcoholate of an alkali metal or an alkaline earth metal with a lithium salt having good solubility.

### CITATION LIST

### PATENT LITERATURE

Patent Literature 1: JP-A-S55-143935
Patent Literature 2: JP-A-S64-034947 Patent Literature 3: JP-H07-133252

### SUMMARY OF INVENTION

### TECHNICAL PROBLEM

However, Patent Literature 1 does not specifically address the transesterification of an alkyl (meth)acrylate with an alcohol compound having a tertiary hydroxyl group to produce the methacrylate ester of the alcohol compound having a tertiary hydroxyl group.

Patent Literature 2 describes that extended reaction time causes esterification to take place also on the tertiary hydroxyl group in 3-methylbutane-1,3-diol and therefore the reaction needs to be terminated before the second esterification occurs on the monoester of 3-methylbutane-1,3-diol. That is, Patent Literature 2 does not explicitly demonstrate conditions which allow the transesterification of an alkyl methacrylate with an alcohol compound having a tertiary hydroxyl group to give the (meth)acrylate ester compound of the alcohol compound having a tertiary hydroxyl group with a high yield.

The present invention is directed to the production of a (meth)acrylate ester compound of an alcohol compound having a tertiary hydroxyl group. An object of the invention is to provide a method for producing a (meth) acrylate ester compound by the transesterification of an alkyl (meth)acrylate with an alcohol compound having a tertiary hydroxyl group so as to esterify all the hydroxyl groups present in the alcohol compound with a high yield or, in a preferred embodiment, a method for esterifying a polyhydric alcohol compound having a tertiary hydroxyl group and also having a primary hydroxyl group and/or a secondary hydroxyl group by one-pot transesterification into a (meth)acrylate ester compound of the polyhydric alcohol.

### SOLUTION TO PROBLEM

An aspect of the invention resides in a method for producing a (meth) acrylate ester compound including a step (I) of transesterifying an alkyl (meth)acrylate with an alcohol compound having a tertiary hydroxyl group using a transesterification catalyst including a complex of iron with a ligand represented by the following general formula (1) or general formula (2), the method being characterized in that the water content in the transesterification reaction system is or has been controlled to not more than 100 ppm.

[Chem. 1]

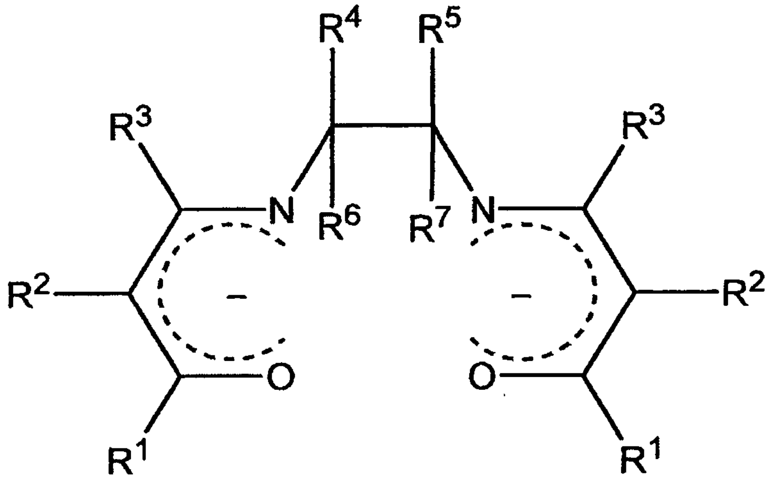

(1)

[Chem. 2]

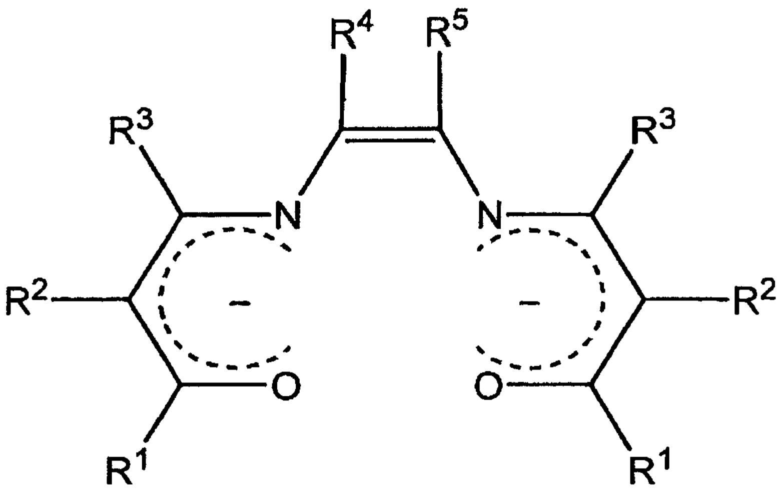

(2)

(In the formula (1) and the formula (2), $R^1$, $R^2$, $R^3$, $R^4$, $R^5$, $R^6$ and $R^7$ are each independently a hydrogen atom, an alkyl group, a monovalent alicyclic group or a monovalent aromatic ring group, and at least one combination of $R^1$ and $R^2$, $R^2$ and $R^3$, and $R^4$ and $R^5$ may form an alicyclic group or an aromatic ring group.)

In the production method of the invention, the transesterification catalyst is preferably used in an amount of 0.1 to 20 mol% in terms of iron atoms relative to the hydroxyl groups of the alcohol compound.

In the production method of the invention, the alcohol compound is preferably a polyhydric alcohol compound having a tertiary hydroxyl group and also having a primary hydroxyl group and/or a secondary hydroxyl group, and the polyhydric alcohol compound is preferably a compound represented by the following general formula (3).

[Chem. 3]

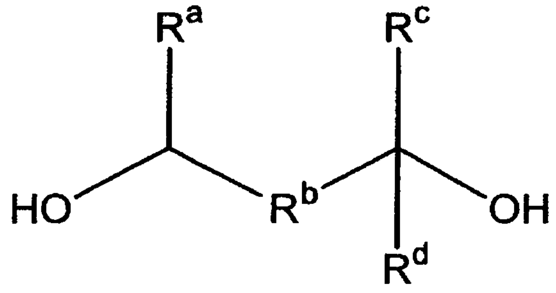

(3)

(In the formula (3), $R^a$ is a hydrogen atom or a monovalent hydrocarbon group having 1 to 4 carbon atoms, $R^b$ is a divalent hydrocarbon group having 1 to 4 carbon atoms, and $R^c$ and $R^d$ are each independently a monovalent hydrocarbon group having 1 to 6 carbon atoms.)

In the production method of the invention, the polyhydric alcohol compound is preferably isoprene glycol.

In the production method of the invention, it is preferable that the raw material alcohol compound having a tertiary hydroxyl group be a polyhydric alcohol compound having a primary hydroxyl group and/or a secondary hydroxyl group, and the (meth) acrylate ester compound obtained be a polyvalent ester compound resulting from the esterification of all the hydroxyl groups present in the polyhydric alcohol compound.

In the production method of the invention, it is preferable that the raw material alcohol compound having a tertiary hydroxyl group be a diol compound represented by the following general formula (3), and the (meth)acrylate ester compound obtained be a diester compound.

[Chem. 4]

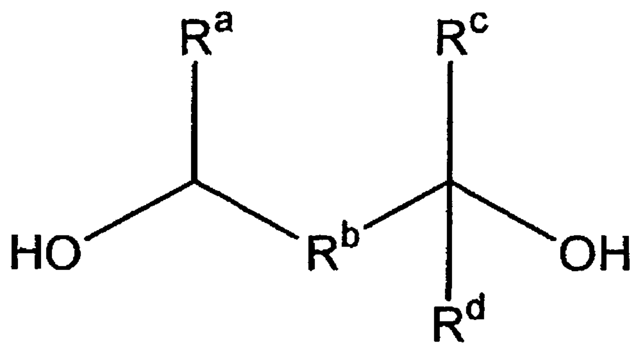

(3)

(In the formula (3), $R^a$ is a hydrogen atom or a monovalent hydrocarbon group having 1 to 4 carbon atoms, $R^b$ is a divalent hydrocarbon group having 1 to 4 carbon atoms, and $R^c$ and $R^d$ are each independently a monovalent hydrocarbon group having 1 to 6 carbon atoms.)

In the production method of the invention, the (meth) acrylate ester compound is preferably di(meth)acrylate ester of isoprene glycol.

The production method of the invention may include a step (II) of dehydrating the transesterification catalyst before use in the step (I).

In the production method of the invention, it is preferable that in the step (II), the transesterification catalyst be dehydrated in the presence of the alcohol compound using a solvent azeotropic with water.

The production method of the invention may include a step (III) of distilling the reaction liquid obtained in the step (I) .

ADVANTAGEOUS EFFECTS OF INVENTION

The production method of the present invention can esterify an alcohol compound having a tertiary hydroxyl group, for example, a polyhydric alcohol compound having a tertiary hydroxyl group and also having a primary and/or secondary hydroxyl group, at all the hydroxyl groups in one pot, thus realizing inexpensive production of (meth)acrylate

ester compounds. Further, isoprene glycol di(meth)acrylate can be obtained with a high yield from isoprene glycol that is an alcohol compound having a tertiary hydroxyl group, and an alkyl (meth)acrylate.

BRIEF DESCRIPTION OF DRAWINGS

Fig. 1 is a diagram illustrating changes with time of the ratios of conversion of isoprene glycol into isoprene glycol monomethacrylate and into isoprene glycol dimethacrylate in the reaction liquid of Example 1.
Fig. 2 is a diagram illustrating changes with time of the ratios of conversion of isoprene glycol into isoprene glycol monomethacrylate and into isoprene glycol dimethacrylate in the reaction liquid of Reference Example 2.
Fig. 3 is a diagram illustrating changes with time of the ratios of conversion of isoprene glycol into isoprene glycol monomethacrylate and into isoprene glycol dimethacrylate in the reaction liquid of Comparative Example 1.

DESCRIPTION OF EMBODIMENTS

The present invention will be described in detail hereinbelow. In the present specification, methacrylate and acrylate are sometimes written collectively as "(meth)acrylate"

A method for producing a (meth)acrylate ester compound according to the present invention includes a step (I) of transesterifying an alkyl (meth)acrylate with an alcohol compound having a tertiary hydroxyl group using a specific transesterification catalyst.

The alcohol compound with a tertiary hydroxyl group that is used in the invention is not particularly limited and may be a known such compound. The alcohol compound having a tertiary hydroxyl group may be a monohydric alcohol compound or a polyhydric alcohol compound such as a dihydric alcohol compound (a diol compound).

Examples of the monohydric alcohol compounds include t-butyl alcohol, t-amyl alcohol and 2-methyl-3-buten-2-ol.

Examples of the polyhydric alcohol compounds include those compounds represented by the following general formula (3) (diol compounds) having a tertiary hydroxyl group and also having a primary hydroxyl group and/or a secondary hydroxyl group.

[Chem. 5]

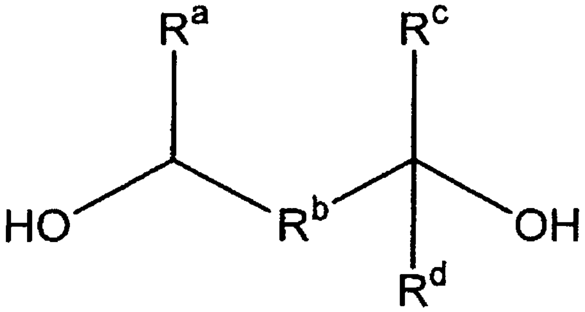

(3)

(In the formula, $R^a$ is a hydrogen atom or a monovalent hydrocarbon group having 1 to 4 carbon atoms, $R^b$ is a divalent hydrocarbon group having 1 to 4 carbon atoms, and $R^c$ and $R^d$ are each independently a monovalent hydrocarbon group having 1 to 6 carbon atoms.)

Examples of the compounds represented by the general formula (3) include isoprene glycol, 4-methyl-2,4-pentanediol, 5-methyl-3,5-hexanediol, 6-methyl-4,6-heptanediol, 7-methyl-5,7-octanediol, 4-methyl-1,4-pentanediol, 5-methyl-1,5-hexanediol, 6-methyl-1,6-heptanediol, 3-methyl-1,3-pentanediol, 3-methyl-1,3-hexanediol, 3-propyl-1,3-hexanediol, 3-ethyl-1,3-heptanediol, 3-methyl-1,3-nonanediol, 4-methyl-1,4-hexanediol, 5-methyl-1,5-heptanediol and 6-methyl-1,6-octanediol.

While the alkyl (meth)acrylate used in the invention is not particularly limited, a compound in which the alkyl group is a lower alkyl group such as methyl, ethyl, n-propyl, iso-propyl, n-butyl, iso-butyl or t-butyl is used because of the ease in distilling away the alcohol resulting from the reaction.

The transesterification catalyst used in the invention is a complex of iron and a ligand represented by the following general formula (1) or general formula (2).

[Chem. 6]

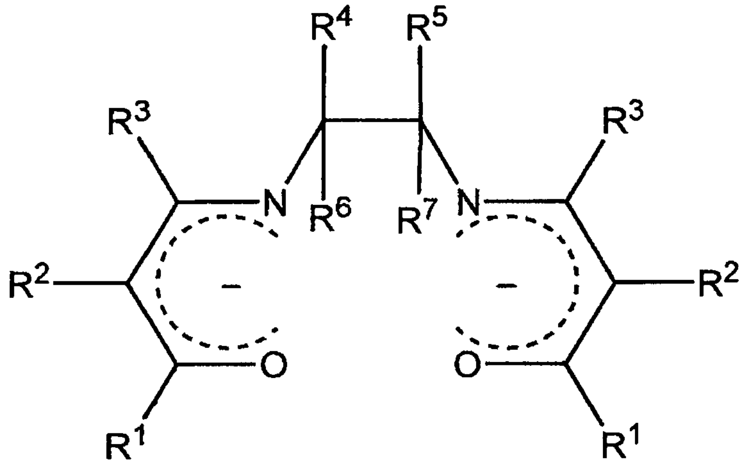

(1)

[Chem. 7]

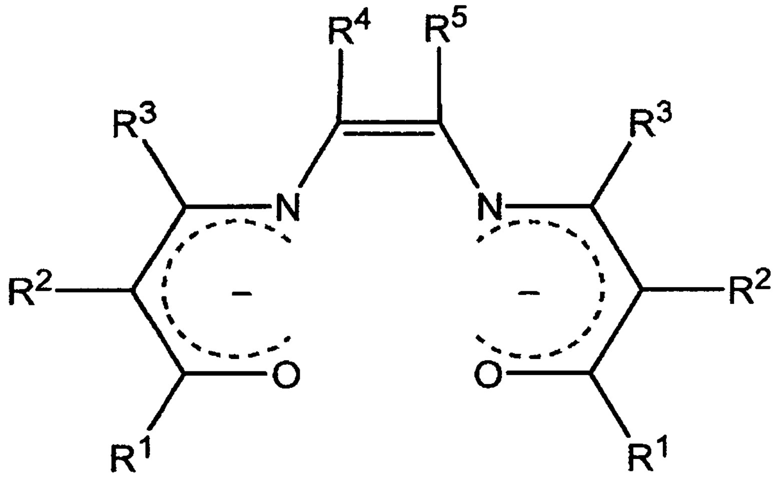

(2)

(In the formula (1) and the formula (2), $R^1$, $R^2$, $R^3$, $R^4$, $R^5$, $R^6$ and $R^7$ are each independently a hydrogen atom, an alkyl group, a monovalent alicyclic group or a monovalent aromatic ring group, and at least one combination of $R^1$ and $R^2$, $R^2$ and $R^3$, and $R^4$ and $R^5$ may form an alicyclic group or an aromatic ring group.)

Examples of the ligands represented by the formula (1) or the formula (2) include N,N'-bis(salicylidene)ethylenediamine, N,N'-bis(salicylidene)orthophenylene ethylenediamine and N,N'-bis(1-methyl-3-oxobutylidene)-4-methylorthophenylene ethylenediamine.

Examples of the iron complexes represented by the formula (1) or the formula (2) include
N,N'-bis(salicylidene)ethylenediamine iron (II),
N,N'-bis(salicylidene)orthophenylene ethylenediamine iron (II) and
N,N'-bis(1-methyl-3-oxobutylidene)-4-methylorthophenylene ethylenediamine iron (II). Of these,
N,N'-bis(salicylidene)ethylenediamine iron (II) is suitably used.

The (meth) acrylate ester compound that is produced by the production method of the invention is preferably an ester compound resulting from the esterification of all the hydroxyl groups in the alcohol compound having a tertiary hydroxyl group. In general, an acid catalyst catalyzes the transesterification of a polyhydric alcohol compound as a raw material which has a tertiary hydroxyl group and also a primary hydroxyl group and/or a secondary hydroxyl group so that the esterification of the primary hydroxyl group and/or the secondary hydroxyl group is accompanied by the dehydration reaction of the tertiary hydroxyl group to cause a risk that the yield of the target compound may be decreased. In contrast, the transesterification catalyst of the invention that includes an iron complex is a neutral catalyst, and does not induce the dehydration reaction of the tertiary hydroxyl group but does allow all the hydroxyl groups to be esterified.

That is, the production method of the invention is suited for esterifying a polyhydric alcohol compound having a tertiary hydroxyl group and also having a primary hydroxyl group and/or a secondary hydroxyl group, at all the hydroxyl groups into a polyvalent ester compound.

Examples of the (meth) acrylate ester compounds, obtained when the alcohol compound having a tertiary hydroxyl group is a monohydric alcohol compound, include t-butyl acrylate, t-butyl methacrylate, t-amyl acrylate, t-amyl methacrylate, 2-methyl-3-butene-2-acrylate and 2-methyl-3-butene-2-methacrylate.

Examples of the (meth) acrylate ester compounds, obtained when the alcohol compound having a tertiary hydroxyl group is, for example, a diol compound that is represented by the general formula (3) below and has a tertiary hydroxyl group and also has a primary hydroxyl group and/or a secondary hydroxyl group, include such diester compounds as isoprene glycol diacrylate, isoprene glycol dimethacrylate, 4-methyl-2,4-pentanediol diacrylate, 4-methyl-2,4-pentanediol dimethacrylate, 5-methyl-3,5-hexanediol diacrylate, 5-methyl-3,5-hexanediol dimethacrylate, 6-methyl-4,6-heptanediol diacrylate, 6-methyl-4,6-heptanediol dimethacrylate, 7-methyl-5,7-octanediol diacrylate, 7-methyl-5,7-octanediol dimethacrylate, 4-methyl-1,4-pentanediol diacrylate, 4-methyl-1,4-pentanediol dimethacrylate, 5-methyl-1,5-hexanediol diacrylate, 5-methyl-1,5-hexanediol dimethacrylate, 6-methyl-1,6-heptanediol diacrylate, 6-methyl-1,6-heptanediol dimethacrylate, 3-methyl-1,3-pentanediol diacrylate, 3-methyl-1,3-pentanediol dimethacrylate, 3-methyl-1,3-hexanediol diacrylate, 3-methyl-1,3-hexanediol dimethacrylate, 3-propyl-1,3-hexanediol diacrylate, 3-propyl-1,3-hexanediol dimethacrylate, 3-ethyl-1,3-heptanediol diacrylate, 3-ethyl-1,3-heptanediol dimethacrylate, 3-methyl-1,3-nonanediol diacrylate, 3-methyl-1,3-nonanediol dimethacrylate, 4-methyl-1,4-hexanediol diacrylate, 4-methyl-1,4-hexanediol dimethacrylate, 5-methyl-1,5-heptanediol diacrylate, 5-methyl-1,5-heptanediol dimethacrylate, 6-methyl-1,6-octanediol diacrylate and 6-methyl-1,6-octanediol dimethacrylate.

[Chem. 8]

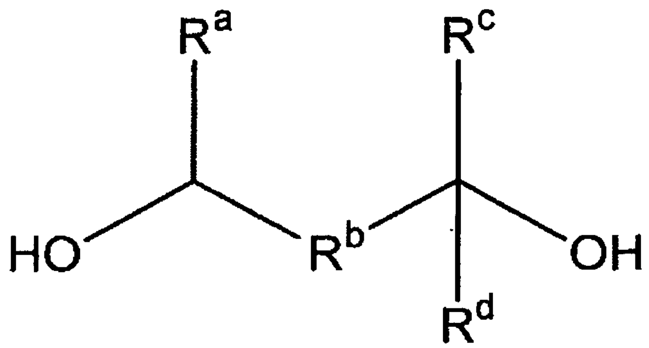

(3)

(In the formula (3), $R^a$ is a hydrogen atom or a monovalent hydrocarbon group having 1 to 4 carbon atoms, $R^b$ is a divalent hydrocarbon group having 1 to 4 carbon atoms, and $R^c$ and $R^d$ are each independently a monovalent hydrocarbon group having 1 to 6 carbon atoms.)

When, for example, the target compound is isoprene glycol dimethacrylate, the raw materials used in the production method of the invention are suitably isoprene glycol as the alcohol compound having a tertiary hydroxyl group, and methyl methacrylate as the alkyl (meth)acrylate.

In the production method of the invention, the rate at which the alcohol compound having a tertiary hydroxyl group is esterified into the target (meth)acrylate ester compound is increased and the yield is enhanced with decreasing water content in the reaction liquid. Thus, the water content in the reaction system in which the transesterification takes place (in the reaction liquid) is not more than 100 ppm. If the water content in the reaction system exceeds 100 ppm, the transesterification activity with respect to the tertiary hydroxyl group is decreased, and the reaction takes a long time and tends to be accompanied by polymerization reaction, resulting in a low yield.

The water content in the reaction system may be maintained low in any manner without limitation as long as the water content in the reaction system can be kept at a low level. In contrast to titanium alcoholate that is a known transesterification catalyst, the iron complex transesterification catalyst of the invention is not decomposed or deactivated by water. Thus, the dehydration of the reaction system may take place before or after the start of the reaction. Some example dehydration techniques are azeotropic dehydration in which water present in the raw materials is removed prior to the reaction using a solvent that is azeotropic with water; the addition of a solvent that is azeotropic with water to the reaction system followed by the reaction while distilling away water as the azeotropic mixture; and adsorption dehydration in which water present in the raw materials is removed using a desiccant such as molecular sieve. In particular, it is preferable that a step (II) of dehydrating the transesterification catalyst before use in the step (I) be added to the production method of the invention. Specifically, the transesterification catalyst is preferably dehydrated in the presence of the

alcohol compound using a solvent azeotropic with water, this manner of dehydration being advantageous in that the thermal history on the alkyl (meth) acrylate is short. The azeotropic solvent may be any of known solvents as long as the solvent does not inhibit reactions such as transesterification, with examples including toluene, xylene, 2-butanone, dioxane, benzene and cyclohexane. When the reaction is performed under total reflux, water may be removed in an adsorptive manner by passing the condensate through a tower filled with a desiccant such as molecular sieve.

In the production method of the invention, the molar ratio of the hydroxyl groups present in the starting alcohol having a tertiary hydroxyl group to the alkyl (meth)acrylate is selected appropriately in consideration of factors such as economic efficiency, boiling point and azeotropic properties, but is usually 1:1 to 1:50, and preferably 1:1 to 1:20.

The amount of the transesterification catalyst used in the production method of the invention is usually 0.1 to 20 mol% in terms of iron atoms relative to the hydroxyl groups of the alcohol compounds having the tertiary hydroxyl groups (when the alcohol compound contains a plurality of hydroxyl groups, the total of the hydroxyl groups), preferably 0.5 to 15 mol%, and more preferably 1 to 10 mol%. If the amount is excessively large, the cost is excessively increased. If the amount is excessively small, the reaction takes too long a time and the productivity tends to be decreased.

In the production method of the invention, the reaction temperature is usually 70 to 150°C, preferably 80 to 120°C, and more preferably 90 to 110°C. The lower the reaction temperature, the longer the reaction time and the lower the productivity. An excessively high reaction temperature increases a risk of simultaneous polymerization reaction. The reaction pressure may be atmospheric pressure or, to facilitate the removal of methanol formed, may be reduced pressure. From the point of view of productivity, the reaction time is usually not more than 200 hours, preferably not more than 150 hours, and more preferably not more than 100 hours.

In the production method of the invention, a reaction solvent may be used. The reaction solvent may be any of known solvents except those solvents which undergo side reactions with the materials such as the alcohol compound or the (meth)acrylate ester compound or which inhibit reactions such as transesterification. The solvent may be selected appropriately in consideration of factors such as azeotropic properties with water and the byproduct alcohol, and reaction temperature. Examples of the reaction solvents include toluene, xylene, 2-butanone, dioxane, benzene and cyclohexane.

The reaction liquid obtained in the step (I) may be subjected to a distillation step (III) to remove undesired components such as the unreacted raw materials. Examples of the distillation methods include thin-film distillation and packed column distillation.

To prevent the simultaneous occurrence of polymerization reaction, the production method of the invention preferably involves the addition of a polymerization inhibitor and/or the introduction of oxygen into the reaction system. The polymerization inhibitor may be any of known substances without limitation, with examples including hydroquinone, hydroquinone monomethyl ether, di-t-butylhydroxytoluene, phenothiazine and N,N'-dinaphthyl-p-phenylenediamine. A single or a combination of such polymerization inhibitors may be used.

## EXAMPLES

The present invention will be described in further detail by presenting Examples and Comparative Example hereinbelow without limiting the scope of the invention to such Examples.

The reaction liquids in Examples and Comparative Example were analyzed in accordance with Test Examples 1 to 3 described below.

### (Test Example 1) Quantitative analysis of reaction liquid

The reaction liquid passed through a filter was analyzed by gas chromatography. Using tridecane as the internal standard, isoprene glycol, isoprene glycol monomethacrylate and isoprene glycol dimethacrylate were quantitatively determined.

### (Gas chromatography conditions)

Chromatograph: GC-2014 (manufactured by Shimadzu Corporation)
Column: DB-1 0.25 mm ϕ × 30 mm, film thickness 0.25 μm (manufactured by Agilent Technologies Japan, Ltd.)
Injection temperature: 280°C
Column temperature: held at 50°C for 5 minutes, increased to 280°C at 10°C/min, and held for 3 minutes.
FID detector temperature: 280°C
Carrier gas: helium, column flow rate 1.5 mL/min
Amount of injection: 0.2 μL

(Test Example 2) Water content

In Examples 1 and 2 and Comparative Example 1, the amount of water in the raw materials that had been fed and the amount of water in the fraction were measured by the Karl Fischer moisture analysis, and the water content in the reaction liquid was determined using the equation below.

Amount of water in raw materials fed (g) = Weight of raw materials fed (g) × Water content in raw materials fed (ppm)

Amount of water in fraction (g) = Weight of fraction (g) × Water content in fraction (ppm)

Water content in reaction liquid (ppm) = {Amount of water in raw materials fed (g) - Amount of water in fraction (g)}/Weight of reaction liquid (g) × 1000000

In Examples 3 and 4, the water content in the reaction liquid was determined by measuring the amount of water in the fraction to be returned to the reaction system through a molecular sieve by the Karl Fischer moisture analysis.

(Conditions of Karl Fischer moisture analysis)

Apparatus: CA-100 (manufactured by Mitsubishi Chemical Corporation)
Anode liquid: AQUAMICRON AX (manufactured by Mitsubishi Chemical Corporation)
Cathode liquid: AQUAMICRON CXU (manufactured by Mitsubishi Chemical Corporation)
Amount of injection: 0.2 g

〈Test Example 3〉 Evaluation of gelation of reaction liquid

The evaluation of the gelation of the reaction liquid indicates whether or not the increase in the viscosity of the reaction liquid will cause a difficulty in recovering the reaction liquid from the reactor after the completion of the reaction or in handling the reaction liquid during distillation. Specifically, the reaction liquid of the system of Reference Example described later was analyzed in accordance with Test Example 1 to determine the ratios of conversion into isoprene glycol monomethacrylate and isoprene glycol dimethacrylate. The total of the conversion ratios measured was compared to the theoretical total conversion into isoprene glycol monomethacrylate and isoprene glycol dimethacrylate taken as 100 mol%, and the difference was obtained as the ratio of decrease. Plotting the ratio of decrease against the reaction time allows for an estimation of the correlation of the reaction time and the ratio of decrease. Based on the reaction times expected in Examples 1 and 2 and Comparative Example 1 described later, the ratios of decrease were calculated and the gelation of the reaction liquids was evaluated. The reaction liquid is easy to handle when the ratio of decrease is less than 20 mol%, and is difficult to handle if the ratio is 20 mol% or above.

(Example 1)

A 50 mL three-necked flask equipped with a reflux column, a dropping funnel, a thermometer, a fraction tray and a drying tube was loaded with 0.64 g (2 mmol) of N,N'-bis(salicylidene)ethylenediamine iron (II), 1.04 g (10 mmol) of isoprene glycol, 40 g (400 mmol) of methyl methacrylate, 0.08 g of phenothiazine and 0.2 g of tridecane. While performing stirring at atmospheric pressure, the flask was placed into an oil bath set at 120°C so that the temperature inside the flask would be 100 to 105°C. The reaction was performed for 15 hours while discharging the fraction recovered in the fraction tray and continuously dropping the same volume of methyl methacrylate as the fraction. The water content

of the reaction liquid during the reaction was 66 ppm. The reaction liquid was sampled at prescribed lapses of time from the start of the reaction (0 hour, 1 hour, 2 hours, 3 hours, 5 hours, 7 hours, 11 hours and 15 hours) . The samples of the reaction liquid were quantitatively analyzed as described in Test Example 1, and the changes with time were tracked, the results being illustrated in Fig. 1.

Reference 〈Example 2〉

The reaction was performed in the same manner as in Example 1, except that the water content of the reaction liquid during the reaction was 585 ppm and the reaction time was 5 hours. The reaction liquid was sampled at prescribed lapses of time from the start of the reaction (0 hour, 1 hour, 2 hours, 3 hours and 5 hours). The samples of the reaction liquid were quantitatively analyzed as described in Test Example 1, and the changes with time were tracked, the results being illustrated in Fig. 2.

(Comparative Example 1)

The reaction was performed in the same manner as in Example 1, except that the water content of the reaction liquid during the reaction was 1818 ppm and the reaction time was 5 hours. The reaction liquid was sampled at prescribed lapses of time from the start of the reaction (0 hour, 1 hour, 2 hours, 3 hours and 5 hours). The samples of the reaction liquid were quantitatively analyzed as described in Test Example 1, and the changes with time were tracked, the results being illustrated in Fig. 3.

The results of Examples 1 and 2 and Comparative Example 1 show that while the rates at which isoprene glycol monomethacrylate was formed from isoprene glycol were similar, the rates of the formation of isoprene glycol dimethacrylate from isoprene glycol monomethacrylate decreased as the water content in the reaction liquid was higher.

From the results of Example 1, the reaction time expected in order to obtain isoprene glycol dimethacrylate with a high yield of at least 99.9% is about 40 hours. The reaction time expected from the results of Example 2 is about 100 hours. In contrast, about 350 hours of reaction time is expected from the results of Comparative Example 1.

(Reference Example)

A 50 mL three-necked flask that had been equipped with a column fitted with a side tube and packed with 20 g of molecular sieve (4A), a condenser, a thermometer and a drying tube was loaded with 0.64 g (2 mmol) of N,N'-bis(salicylidene)ethylenediamine iron (II), 1.04 g (10 mmol) of isoprene glycol, 40 g (400 mmol) of methyl methacrylate, 0.08 g of phenothiazine and 0.2 g of tridecane. While performing stirring at atmospheric pressure, the flask was placed into an oil bath set at 120°C so that the temperature inside the flask would be 100 to 105°C. The reaction was performed for 11 hours while totally refluxing the distilled fraction back to the reaction system through the molecular sieve. The reaction liquid was sampled at prescribed lapses of time from the start of the reaction (3 hours, 5 hours, 7 hours, 9 hours and 11 hours). The gelation of the samples of the reaction liquid was evaluated as described in Test Example 3, the results being described in Table 1.

[Table 1]

| Reaction time (hours) | | 3 | 5 | 7 | 9 | 11 |
|---|---|---|---|---|---|---|
| Conversion ratios (mol%) | Isoprene glycol monomethacrylate | 50.5 | 14.0 | 4.0 | 0.4 | 0.0 |
| | Isoprene glycol dimethacrylate | 49.5 | 86.0 | 94.4 | 95.8 | 94.9 |
| Ratio of decrease (mol%) | | 0 | 0 | 1. 6 | 3.8 | 5.1 |

As described in the results of Reference Example, the ratios of conversion of isoprene glycol monomethacrylate and isoprene glycol dimethacrylate started to fall after a lapse of 7 hours from the start of the reaction, and the ratio of decrease after 11 hours from the start of the reaction was about 5 mol%. The ratio of decrease of isoprene glycol monomethacrylate and isoprene glycol dimethacrylate calculated from this result and the expected reaction time described hereinabove is approximately 14 mol% in Example 1 (about 40 hours) and is approximately 19 mol% in Example 2 (about 100 hours), whereas the ratio of decrease in Comparative Example 1 (about 350 hours) is calculated to be approximately 28 mol%. Accordingly, gelation is highly likely to occur in Comparative Example 1.

(Example 3)

A 50 mL three-necked flask that had been equipped with a column fitted with a side tube and packed with 30 g of molecular sieve (4A), a condenser, a thermometer and a drying tube was loaded with 0.51 g (1.6 mmol) of N,N'-bis(salicylidene)ethylenediamine iron (II), 4.16 g (40 mmol) of isoprene glycol and 35 g of toluene. While performing stirring at atmospheric pressure, the flask was placed into an oil bath set at 130°C so that the temperature inside the flask would be 110 to 112°C. The distilled fraction was totally refluxed through the molecular sieve for 4 hours, and thereby N,N'-bis(salicylidene)ethylenediamine iron (II) was dissolved. A catalyst-isoprene glycol-toluene solution was thus obtained. Toluene was distilled away from the toluene solution under reduced pressure, and a black red catalyst-isoprene glycol solution was obtained.

A 100 mL three-necked flask that had been equipped with a column fitted with a side tube and packed with 30 g of molecular sieve (4A), a condenser, a thermometer and a drying tube was loaded with the whole amount of the catalyst-isoprene glycol solution obtained above, 80 g (800 mmol) of methyl methacrylate, 0.16 g of phenothiazine and 0.8 g of tridecane. While performing stirring at atmospheric pressure, the flask was placed into an oil bath set at 120°C so that the temperature inside the flask would be 100 to 105°C. The reaction was performed for 7 hours while totally refluxing the distilled fraction back to the reaction system through the molecular sieve. The water content in the reaction liquid during the reaction was 0.1 ppm. The reaction liquid was sampled at prescribed lapses of time from the start of the reaction (0 hour, 1 hour, 2 hours, 3 hours, 5 hours and 7 hours). The samples of the reaction liquid were quantitatively analyzed as described in Test Example 1, and the changes with time in the ratio of conversion (mol%) from isoprene glycol to isoprene glycol dimethacrylate were tracked, the results being described in Table 2.

(Example 4)

A 100 mL three-necked flask that had been equipped with a column fitted with a side tube and packed with 20 g of molecular sieve (4A), a condenser, a thermometer and a drying tube was loaded with 0.51 g (1.6 mmol) of N,N'-bis(salicylidene)ethylenediamine iron (II), 4.16 g (40 mmol) of isoprene glycol, 80 g (800 mmol) of methyl methacrylate, 0.16 g of phenothiazine and 0.8 g of tridecane. While performing stirring at atmospheric pressure, the flask was placed into an oil bath set at 120°C so that the temperature inside the flask would be 100 to 105°C. The reaction was performed for 7 hours while totally refluxing the distilled fraction back to the reaction system through the molecular sieve. The water content in the reaction liquid during the reaction was 0.3 ppm. The reaction liquid was sampled at prescribed lapses of time from the start of the reaction (0 hour, 1 hour, 2 hours, 3 hours, 5 hours and 7 hours). The samples of the reaction liquid were quantitatively analyzed as described in Test Example 1, and the changes with time in the ratio of conversion (mol%) from isoprene glycol to isoprene glycol dimethacrylate were tracked, the results being described in Table 2.

[Table 2]

| | Reaction time (hours) | 0 | 1 | 2 | 3 | 5 | 7 |
|---|---|---|---|---|---|---|---|
| Ex. 3 | Conversion ratio | 0.0 | 11.0 | 27.4 | 45.9 | 68.2 | 87.5 |
| Ex. 4 | (mol%) | 0.0 | 6.4 | 20.6 | 36.2 | 64.5 | 80.6 |

The results of Examples 3 and 4 show that the azeotropic dehydration pretreatment of N,N'-bis(salicylidene)ethylenediamine iron (II) and isoprene glycol results in a further increase in the rate of the formation of isoprene glycol dimethacrylate in the transesterification reaction.

Extended reaction time decreases productivity and increases costs. Further, the yield may be decreased due to a high risk of the reaction being accompanied by polymerization reaction of the product with methyl methacrylate. Thus, as apparent from the results of Examples and Comparative Example, the transesterification of an alkyl (meth)acrylate and an alcohol compound having a tertiary hydroxyl group needs to be completed efficiently in a short time by regulating the water content in the transesterification reaction system (the reaction liquid) to the low range specified in the present invention.

INDUSTRIAL APPLICABILITY

The production method of the present invention can efficiently produce, in one pot and at low cost, a (meth)acrylate ester compound of an alcohol compound having a tertiary hydroxyl group, preferably, of a polyhydric alcohol compound having a tertiary hydroxyl group and also having a primary hydroxyl group and/or a secondary hydroxyl group. Thus, the production method of the invention is useful for the mass production of a (meth)acrylate ester of an alcohol compound having a tertiary hydroxyl group on an industrial scale.

## Claims

1. A method for producing a (meth)acrylate ester compound comprising a step (I) of transesterifying an alkyl (meth)acrylate with an alcohol compound having a tertiary hydroxyl group using a transesterification catalyst including a complex of iron with a ligand represented by the following general formula (1) or general formula (2), the water content in the transesterification reaction system being not more than 100 ppm;

[Chem. 1]

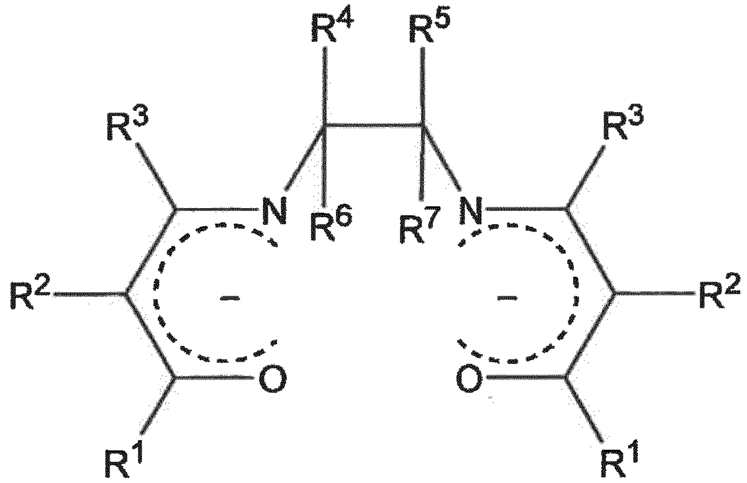

(1)

[Chem. 2]

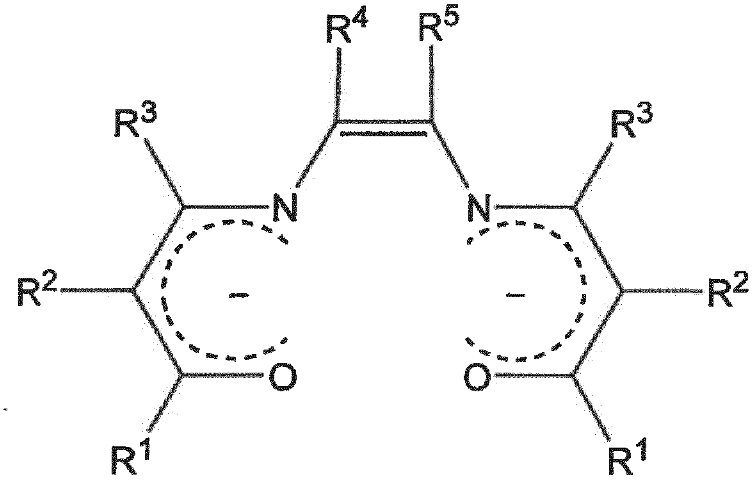

(2)

(in the formula (1) and the formula (2), $R^1$, $R^2$, $R^3$, $R^4$, $R^5$, $R^6$ and $R^7$ are each independently a hydrogen atom, an alkyl group, a monovalent alicyclic group or a monovalent aromatic ring group, and at least one combination of $R^1$ and $R^2$, $R^2$ and $R^3$, and $R^4$ and $R^5$ may form an alicyclic group or an aromatic ring group).

2. The production method according to Claim 1, wherein the transesterification catalyst is used in an amount of 0.1 to 20 mol% in terms of iron atoms relative to the hydroxyl groups of the alcohol compound.

3. The production method according to Claim 1 or 2, wherein the alcohol compound is a polyhydric alcohol compound having a tertiary hydroxyl group and also having a primary hydroxyl group and/or a secondary hydroxyl group.

4. The production method according to any of Claims 1 to 3, wherein the polyhydric alcohol compound is a compound represented by the following general formula (3):

[Chem. 3]

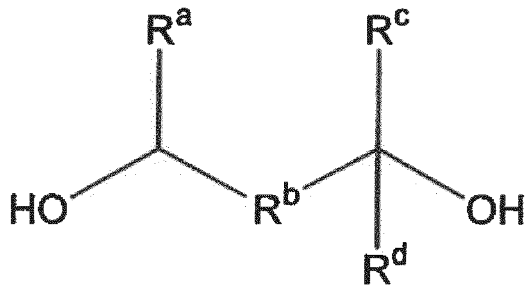

(3)

(in the formula (3), $R^a$ is a hydrogen atom or a monovalent hydrocarbon group having 1 to 4 carbon atoms, $R^b$ is a divalent hydrocarbon group having 1 to 4 carbon atoms, and $R^c$ and $R^d$ are each independently a monovalent hydrocarbon group having 1 to 6 carbon atoms).

**5.** The production method according to any of Claims 1 to 4, wherein the polyhydric alcohol compound is isoprene glycol.

**6.** The production method according to any of Claims 1 to 5, wherein the raw material alcohol compound having a tertiary hydroxyl group is a polyhydric alcohol compound having a primary hydroxyl group and/or a secondary hydroxyl group, and the (meth)acrylate ester compound obtained is a polyvalent ester compound resulting from the esterification of all the hydroxyl groups present in the polyhydric alcohol compound.

**7.** The production method according to any of Claims 1 to 6, wherein the raw material alcohol compound having a tertiary hydroxyl group is a diol compound represented by the following general formula (3), and the (meth)acrylate ester compound obtained is a diester compound;

[Chem. 4]

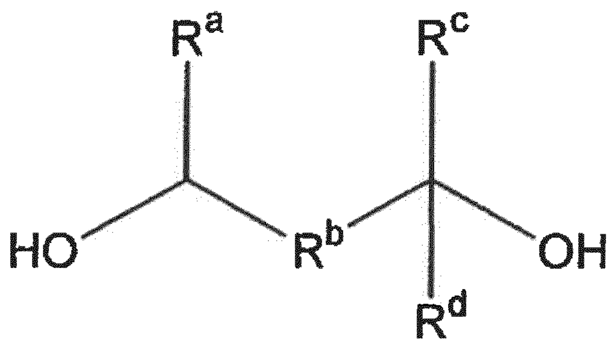

(3)

(in the formula (3), $R^a$ is a hydrogen atom or a monovalent hydrocarbon group having 1 to 4 carbon atoms, $R^b$ is a divalent hydrocarbon group having 1 to 4 carbon atoms, and $R^c$ and $R^d$ are each independently a monovalent hydrocarbon group having 1 to 6 carbon atoms).

**8.** The production method according to any of Claims 1 to 7, wherein the (meth)acrylate ester compound is di(meth)acrylate ester of isoprene glycol.

**9.** The production method according to any of Claims 1 to 8, further comprising a step (II) of dehydrating the transesterification catalyst before use in the step (I).

**10.** The production method according to Claim 9, wherein in the step (II), the transesterification catalyst is dehydrated in the presence of the alcohol compound using a solvent azeotropic with water.

**11.** The production method according to any of Claims 1 to 10, further comprising a step (III) of distilling the reaction liquid obtained in the step (I).

## Patentansprüche

1. Verfahren zur Herstellung einer (Meth)acrylatesterverbindung, umfassend einen Schritt (I) des Umesterns eines Alkyl(meth)acrylats mit einer Alkoholverbindung mit einer tertiären Hydroxylgruppe unter Verwendung eines Umesterungskatalysators, der einen Komplex von Eisen mit einem Liganden, dargestellt durch die nachstehende allgemeine Formel (1) oder die allgemeine Formel (2), beinhaltet, wobei der Wassergehalt im Umesterungsreaktionssystem nicht mehr als 100 ppm beträgt;

[Chem. 1]

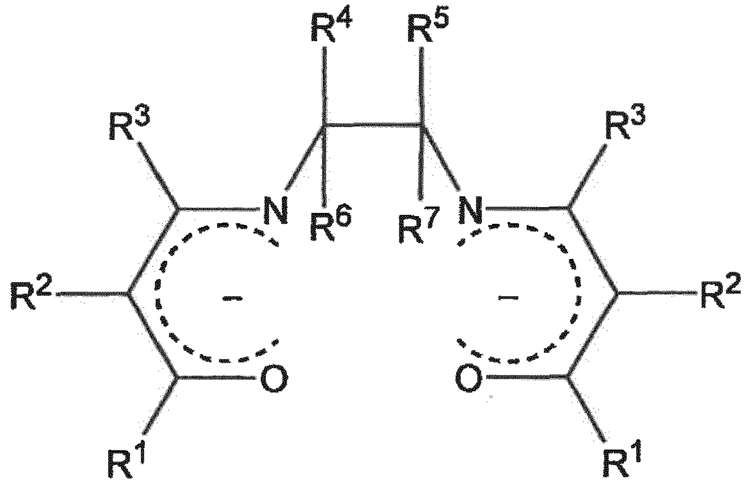

(1)

[Chem. 2]

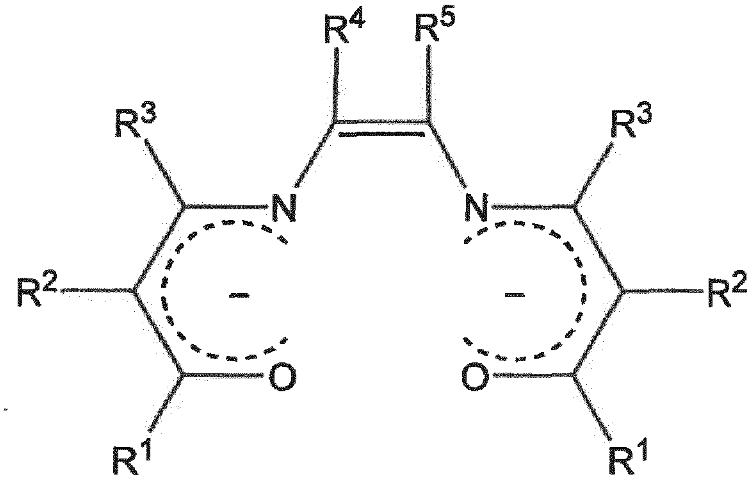

(2)

(in der Formel (1) und der Formel (2) sind $R^1$, $R^2$, $R^3$, $R^4$, $R^5$, $R^6$ und $R^7$ jeweils unabhängig ein Wasserstoffatom, eine Alkylgruppe, eine monovalente alicyclische Gruppe oder eine monovalente aromatische Ringgruppe, und mindestens eine Kombination von $R^1$ und $R^2$, $R^2$ und $R^3$, und $R^4$ und $R^5$ kann eine alicyclische Gruppe oder eine aromatische Ringgruppe bilden).

2. Herstellungsverfahren nach Anspruch 1, wobei der Umesterungskatalysator in einer Menge von 0,1 bis 20 Mol-%, ausgedrückt als Eisenatome, bezogen auf die Hydroxylgruppen der Alkoholverbindung, verwendet wird.

3. Herstellungsverfahren nach Anspruch 1 oder 2, wobei die Alkoholverbindung eine mehrwertige Alkoholverbindung mit einer tertiären Hydroxylgruppe und auch mit einer primären Hydroxylgruppe und/oder einer sekundären Hydroxylgruppe ist.

4. Herstellungsverfahren nach einem der Ansprüche 1 bis 3, wobei die mehrwertige Alkoholverbindung eine Verbindung, dargestellt durch die nachstehende allgemeine Formel (3), ist:

[Chem. 3]

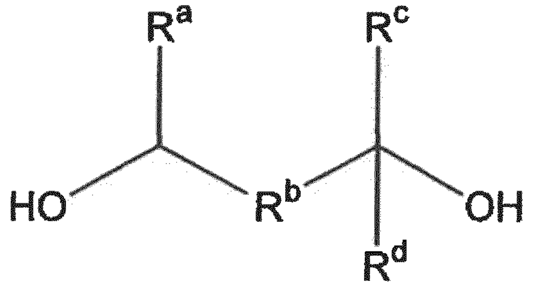

(3)

(in der Formel (3) ist $R^a$ ein Wasserstoffatom oder eine einwertige Kohlenwasserstoffgruppe mit 1 bis 4 Kohlenstoffatomen, $R^b$ ist eine zweiwertige Kohlenwasserstoffgruppe mit 1 bis 4 Kohlenstoffatomen und $R^c$ und $R^d$ sind jeweils unabhängig eine einwertige Kohlenwasserstoffgruppe mit 1 bis 6 Kohlenstoffatomen).

5. Herstellungsverfahren nach einem der Ansprüche 1 bis 4, wobei die mehrwertige Alkoholverbindung Isoprenglykol ist.

6. Herstellungsverfahren nach einem der Ansprüche 1 bis 5, wobei die Ausgangsmaterial-Alkoholverbindung mit einer tertiären Hydroxylgruppe eine mehrwertige Alkoholverbindung mit einer primären Hydroxylgruppe und/oder einer sekundären Hydroxylgruppe ist und die erhaltene (Meth)acrylatesterverbindung eine mehrwertige Esterverbindung ist, die aus der Veresterung aller in der mehrwertigen Alkoholverbindung vorhandenen Hydroxylgruppen resultiert.

7. Herstellungsverfahren nach einem der Ansprüche 1 bis 6, wobei die Rohmaterial-Alkoholverbindung mit einer tertiären Hydroxylgruppe eine Diolverbindung, dargestellt durch die nachstehende allgemeine Formel (3), ist und die erhaltene (Meth)acrylatesterverbindung eine Diesterverbindung ist;

[Chem. 4]

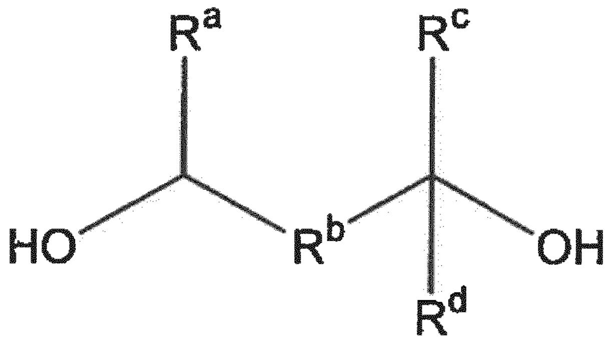

(3)

(in der Formel (3) ist $R^a$ ein Wasserstoffatom oder eine einwertige Kohlenwasserstoffgruppe mit 1 bis 4 Kohlenstoffatomen, $R^b$ ist eine zweiwertige Kohlenwasserstoffgruppe mit 1 bis 4 Kohlenstoffatomen und $R^c$ und $R^d$ sind jeweils unabhängig eine einwertige Kohlenwasserstoffgruppe mit 1 bis 6 Kohlenstoffatomen).

8. Herstellungsverfahren nach einem der Ansprüche 1 bis 7, wobei die (Meth)acrylatesterverbindung Di(meth)acrylatester von Isoprenglykol ist.

9. Herstellungsverfahren nach einem der Ansprüche 1 bis 8, weiter umfassend einen Schritt (II) des Dehydrierens des Umesterungskatalysators vor der Verwendung in dem Schritt (I).

10. Herstellungsverfahren nach Anspruch 9, wobei in dem Schritt (II) der Umesterungskatalysator in Gegenwart der Alkoholverbindung unter Verwendung eines mit Wasser azeotropen Lösungsmittels dehydriert wird.

11. Herstellungsverfahren nach einem der Ansprüche 1 bis 10, weiter umfassend einen Schritt (III) des Destillierens der in dem Schritt (I) erhaltenen Reaktionslösung.

## Revendications

1. Procédé de production d'un composé ester (méth)acrylate, comprenant une étape (I) de transestérification d'un (méth)acrylate d'alkyle avec un composé alcool ayant un groupe hydroxyle tertiaire au moyen d'un catalyseur de transestérification comprenant un complexe du fer avec un ligand représenté par la formule générale (1) ou la formule générale (2) suivante, la teneur en eau dans le système réactionnel de transestérification n'étant pas supérieure à 100 ppm ;

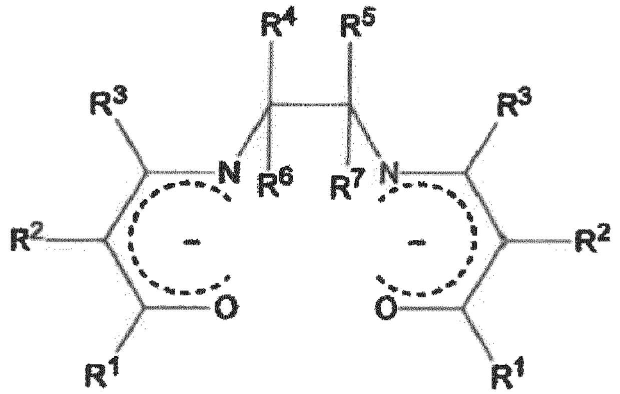

(1)

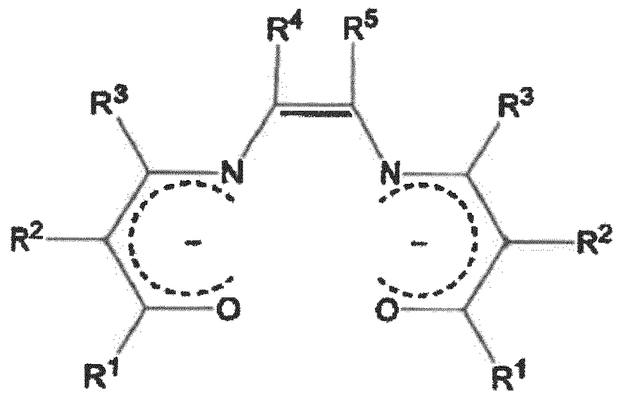

(2)

dans la formule (1) et la formule (2), $R^1$, $R^2$, $R^3$, $R^4$, $R^5$, $R^6$ et $R^7$ étant chacun indépendamment, un atome d'hydrogène, un groupe alkyle, un groupe alicyclique monovalent ou un groupe aromatique monovalent, et au moins une combinaison de $R^1$ et $R^2$, $R^2$ et $R^3$, et $R^4$ et $R^5$ peut former un groupe alicyclique ou un groupe aromatique cyclique.

2. Procédé de production selon la revendication 1, où le catalyseur de transestérification est utilisé en une quantité allant de 0,1 à 20% en moles en termes d'atomes de fer par rapport aux groupes hydroxyle du composé alcool.

3. Procédé de production selon la revendication 1 ou 2, où le composé alcool est un composé alcool polyhydroxylé ayant un groupe hydroxyle tertiaire et ayant également, un groupe hydroxyle primaire et/ou un groupe hydroxyle secondaire.

4. Procédé de production selon l'une quelconque des revendications 1 à 3, où le composé alcool polyhydroxylé est un composé représenté par la formule générale (3) :

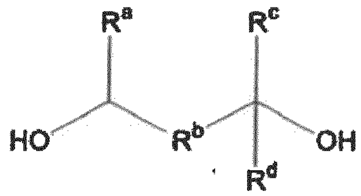

(3)

dans la formule (3), $R^a$ est un atome d'hydrogène ou un groupe hydrocarboné monovalent ayant 1 à 4 atomes de carbone, $R^b$ est un groupe hydrocarboné bivalent ayant 1 à 4 atomes de carbone, et $R^c$ et $R^d$ sont chacun indépendamment, un groupe hydrocarboné monovalent ayant 1 à 6 atomes de carbone.

**5.** Procédé de production selon l'une quelconque des revendications 1 à 4, où le composé alcool polyhydroxylé est l'isoprèneglycol.

**6.** Procédé de production selon l'une quelconque des revendications 1 à 5, où la matière première composé alcool ayant un groupe hydroxyle tertiaire est un composé alcool polyhydroxylé ayant un groupe hydroxyle primaire et/ou un groupe hydroxyle secondaire, et le composé ester (méth)acrylate obtenu est un composé ester polyvalent résultant de l'estérification de tous les groupes hydroxyle présents sur le composé alcool polyhydroxylé.

**7.** Procédé de production selon l'une quelconque des revendications 1 à 6, où la matière première composé alcool ayant un groupe hydroxyle tertiaire est un composé diol représenté par la formule générale (3) suivante, et le composé ester (méth)acrylate obtenu est un composé diester ;

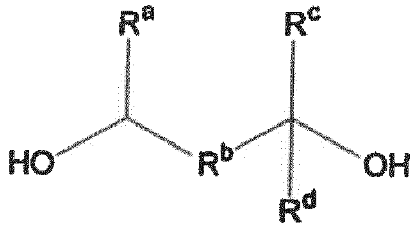

(3)

dans la formule (3), $R^a$ est un atome d'hydrogène ou un groupe hydrocarboné monovalent ayant 1 à 4 atomes de carbone, $R^b$ est un groupe hydrocarboné bivalent ayant 1 à 4 atomes de carbone, et $R^c$ et $R^d$ sont chacun indépendamment, un groupe hydrocarboné monovalent ayant 1 à 6 atomes de carbone.

**8.** Procédé de production selon l'une quelconque des revendications 1 à 7, où le composé ester (méth)acrylate est un ester di(méth)acrylate de l'isoprèneglycol.

**9.** Procédé de production selon l'une quelconque des revendications 1 à 8, comprenant en outre, une étape (II) de déshydratation du catalyseur de transestérification avant son utilisation dans l'étape (I).

**10.** Procédé de production selon la revendication 9, où dans l'étape (II), le catalyseur de transestérification est déshydraté en présence du composé alcool à l'aide d'un solvant formant azéotrope avec l'eau.

**11.** Procédé de production selon l'une quelconque des revendications 1 à 10, comprenant en outre, une étape (III) de distillation du liquide réactionnel obtenu à l'étape (I).

[FIGURE 1]

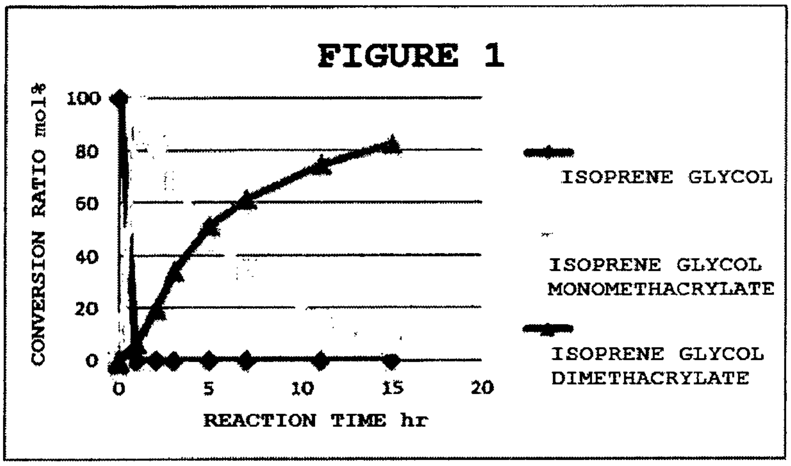


[FIGURE 2]

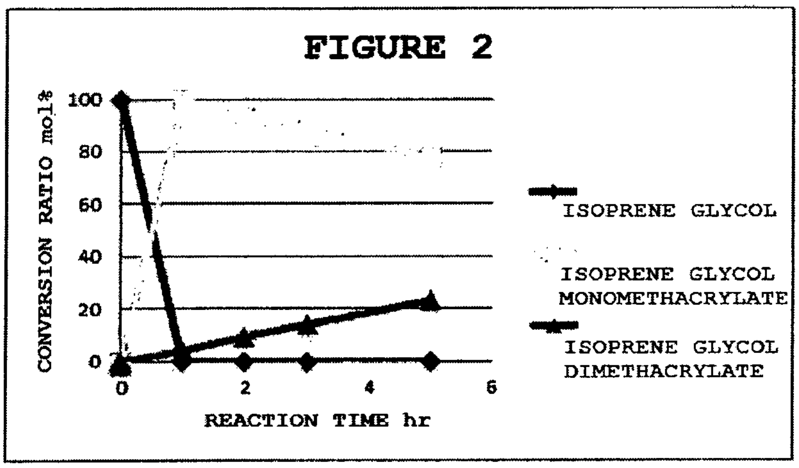


[FIGURE 3]

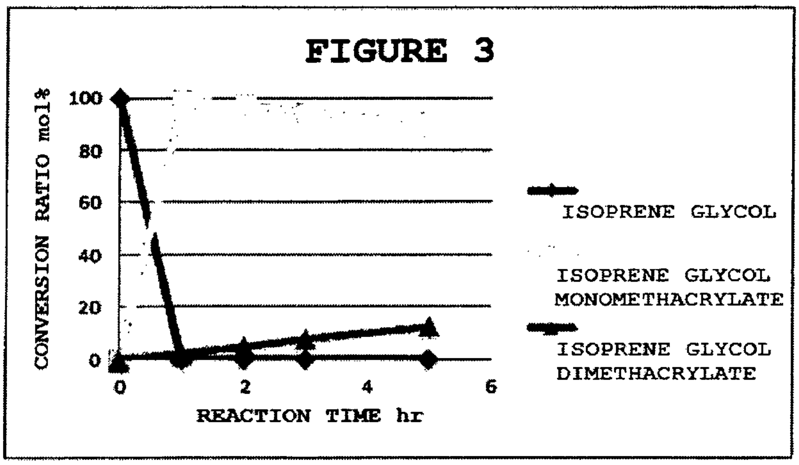

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP S55143935 A
- JP S64034947 A
- JP H07133252 B